(19) **Europäisches Patentamt**

**European Patent Office**

**Office européen des brevets**

(11) **EP 1 140 744 B1**

(12) **EUROPEAN PATENT SPECIFICATION**

(45) Date of publication and mention
of the grant of the patent:
**26.03.2003 Bulletin 2003/13**

(51) Int Cl.[7]: **C07C 11/02**, C07C 5/48,
C10G 11/20

(21) Application number: **99959585.3**

(86) International application number:
**PCT/GB99/04193**

(22) Date of filing: **13.12.1999**

(87) International publication number:
**WO 00/037399 (29.06.2000 Gazette 2000/26)**

(54) **PROCESS FOR THE PRODUCTION OF OLEFINS**

VERFAHREN ZUR HERSTELLUNG VON OLEFINEN

PROCEDE DE PRODUCTION D'OLEFINES

(84) Designated Contracting States:
**AT BE CH CY DE DK ES FI FR GB GR IE IT LI LU MC NL PT SE**
Designated Extension States:
**RO**

(30) Priority: **22.12.1998 GB 9828337**

(43) Date of publication of application:
**10.10.2001 Bulletin 2001/41**

(73) Proprietor: **BP Chemicals Limited**
**London EC2M 7BA (GB)**

(72) Inventors:
• **BURNS, Andrew Lindsay**
**Kinross KY13 0PU (GB)**
• **GRIFFITHS, David Charles**
**Esher Surrey KT10 0HU (GB)**

(74) Representative: **Brooke, Caron et al**
**BP International Limited,**
**Patents & Agreements,**
**Chertsey Road**
**Sunbury-on-Thames, Middlesex TW16 7LN (GB)**

(56) References cited:
**EP-A- 0 332 289**          **WO-A-94/04632**

**Description**

The present invention relates to a process for the production of olefins and in particular to the production of ethene and propene.

A known commercial route for the production of olefins is via steam cracking of paraffinic hydrocarbons. Steam cracking involves the pyrolysis of the hydrocarbons at high temperature. The heat required for the pyrolysis is provided by indirect heat transfer from a radiant furnace. Alternatively, olefins can be prepared by cracking a paraffinic feed wherein the heat required for pyrolysis is provided by the partial combustion of the hydrocarbon feedstock. This latter process is essentially an integrated process comprising the exothermic partial combustion and endothermic cracking of hydrocarbons and is known as "autothermal cracking". The process is disclosed in EP-A-0332289.

A typical product stream from the autothermal cracking of paraffinic hydrocarbons is ethene, propene, butene, higher olefins and carbon monoxide with small amounts of methane, acetylenes, aromatic hydrocarbons, water, hydrogen and carbon dioxide. Ethene and propene are typically the most valuable products as these olefins are often used as feedstock for many processes. There is however a need to separate the ethene and/or propene from the product stream and conventionally this has been accomplished through the use of cryogenic distillation. Such processes are multi-stage typically comprising (1) a first step of separating and removing methane, carbon monoxide and hydrogen via a demethaniser; (2) a second step of separating the $C_2$ hydrocarbons from the heavier hydrocarbons; (3) a third step of hydrogenating the $C_2$ hydrocarbons, wherein acetylene is converted to ethene, separating the ethene from ethane whilst simultaneously recovering propene from the $C_3$ hydrocarbons in a similar manner. In such processes the cryogenic separation is expensive.

We have now developed an integrated process for the production and isolation of olefins which avoids the use of the most expensive and energy intensive cryogenic stages as required in the conventional art.

Accordingly, the present invention provides a process for the production and recovery of olefins from a hydrocarbon feed comprising one or more paraffins, said process comprising the steps of:

(a) partially combusting a mixture of the hydrocarbon feed and a molecular oxygen-containing gas in a reaction chamber, said mixture having a ratio of hydrocarbon to oxygen of greater than the stoichiometric ratio required for complete combustion to carbon dioxide and water, to produce a product stream comprising ethene and propene,
(b) contacting said product stream with a solution of a metallic salt capable of selectively chemically absorbing ethene and propene to produce a chemically absorbed olefin rich liquid stream.
(c) recovering said ethene and/or propene from the metallic salt.

The present invention provides an effective and efficient means of producing and isolating the olefins particularly ethene and propene from the product stream.

The feedstock in the present process is a paraffin-containing hydrocarbon. The feedstock may comprise gaseous hydrocarbons, liquid hydrocarbons or a mixture thereof. Suitable gaseous hydrocarbons are ethane, propane, butane, or mixtures thereof. Suitable liquid hydrocarbons include paraffin containing hydrocarbons such as naphtha, gas oil, vacuum gas oil, refinery residues, atmospheric residues, vacuum residues or mixtures of liquid hydrocarbons as found in crude or fuel oils.

Additional feed components may be included with the hydrocarbon feed if so desired. In particular, where the feed is a liquid hydrocarbon methane, ethane, propane, butane and mixtures thereof may be cofed into the reactor. Additional gases such as carbon dioxide, hydrogen, nitrogen, carbon monoxide or steam may also be co-fed into the feedstock stream.

Where the hydrocarbon feed is a liquid hydrocarbon, the feed may be passed directly into the reactor in the liquid state. The feed may be pre-heated if desired. Suitably, the additional co-fed gases such as hydrogen are mixed with the liquid feed. Suitably, the mixture is introduced into the reaction chamber as a fine spray of droplets such that vaporisation and homogeneous mixing may result. Any suitable means may be used for providing a fine spray of liquid. Suitably, the liquid may be passed through a nozzle. The size of the liquid droplets is suitably small to provide a fine spray of liquid. It will of course be understood that small droplets provide a more efficient vaporisation.

The hydrocarbon feed is mixed with a molecular oxygen-containing gas. Suitably, the gas is oxygen, optionally diluted with an inert gas such as nitrogen. It is preferred to pre-mix the oxygen-containing gas and the paraffinic feed prior to entering the reaction chamber. Suitably the amount of oxygen required for the process of the present invention is from 2 to 20%, preferably 4 to 20% of the amount required for complete combustion to carbon dioxide and water.

Preferably, the velocity of the hydrocarbon and oxygen-containing gas mixture entering the reaction chamber is maintained at a value above the burning velocity of the feed mixture. This is especially applicable to flammable mixtures i.e. a feed mixture which can propagate a flame freely within a limited range of compositions. It will of course be understood by the person skilled in the art that the gas velocity will vary for different hydrocarbon feeds, pre-heat temperature and stoichiometry. Typically, the velocity of the feed mixture may be suitably greater than $0.5 ms^{-1}$, preferably greater than

$1ms^{-1}$. For the purposes of the present invention, velocity is the velocity of the gas through the inlet zone and burning velocity is defined as the measure of unburnt gas velocity at the point of first temperature rise in a flame, perpendicular to the flame front.

The process may be carried in the presence or absence of a catalyst capable of supporting combustion beyond the normal fuel rich limit of flammability. Preferably the process is carried out in the presence of a catalyst. The principal role of the catalyst is to stabilise partial combustion of the mixture which otherwise would not be flammable.

Suitably, the catalyst comprises a supported Group VIII metal. Preferably, the metal is selected from platinum or palladium or a mixture thereof. Suitably, the catalyst comprises from 0.1 to 5%, preferably 0.25 to 2 wt% especially 0.5 to 1 wt % of metal. There is a wide range of support materials available, all of which are well known to the person skilled in the art. The preferred support for the catalyst used in the process of the present invention is alumina. The support material may be in the form of spheres or other granular shapes. The support may also be in the form of a thin layer or wash coat on another substrate. The substrate may be a substantially continuous multi-channel ceramic structure such as a foam or a regular channelled monolith. Metallic substrates may also be used. The preferred wash coat/substrate combination is a alpha alumina coated alpha alumina foam.

The catalyst may be prepared by loading the support with a mixture of platinum and/or palladium by conventional methods well known to those skilled in the art. The resulting compound is then heat treated to $1200°C$ before use in the process of the present invention. Optionally, the compound may be reduced prior to the use as a catalyst.

The hydrocarbon feed and the oxygen-containing gas may be introduced into the reaction chamber under a gas hourly space velocity of suitably greater than 10,000 hour $^{-1}$, preferably, the gas hourly space velocity is greater than 20,000 hour$^{-1}$, most preferably greater than 100,000 hour$^{-1}$. It will of course be understood that the optimum gas hourly space velocity will depend upon the feed and the pressure. For the purposes of the present invention, gas hourly space velocity is defined as:

$$GHSV = \frac{\text{volume of total feed at NTP}}{\text{Time x volume of catalyst bed}} \quad h^{-1}$$

Where the process is operated in the absence of a catalyst, it is necessary to pre-heat the reactants and some additional heat may need to be supplied to the reaction zone in order to stabilise the reaction. This additional heat may be supplied from an external furnace or by appropriate utilisation of the heat of the reactor products. It will be understood that the degree of additional heat required will depend upon the nature of the hydrocarbon, the stoichiometry and gas velocity in the reaction zone.

The process may be suitably carried out at a temperature of from 600 to $1200°C$, preferably from 700 to $1100°C$, most preferably from 750 to $1050°C$.

The process may be carried out under atmospheric pressure or elevated pressure. Where it is desired to use elevated pressure, pressures of up to 30 bara may be used. Examples of suitable pressures are from 1 to 10 bara, preferably, 1 to 5 bara.

Where the process is carried out under elevated pressure, the reaction products may be quenched as they emerge from the reaction chamber to avoid further reactions taking place. The quenching step is particularly suitable to the production of olefins. The reaction product is quenched within 50 milliseconds from formation. It will of course be understood that the time required between product formation and the act of quenching will depend upon the reaction conditions e.g. temperature and pressure.

The products may be quenched using rapid heat exchangers of the type familiar in steam cracking technology. Either in addition to, or instead of the indirect heat exchangers, a direct quench may be employed. Suitable quenching media include water, steam and hydrocarbons such as ethane or naphtha. At the aforementioned temperature and pressure, some of the hydrocarbon quenching media may be cracked to provide additional olefin products in the effluent stream. Such hydrocarbon quenching media are referred to as reactive quenching media.

The amount of quenching media and choice of media which may be usefully employed will depend upon the temperature of the product stream. Optionally, a second quenching fluid such as water may be employed if a hydrocarbon fluid is utilised in the process.

The process may be carried out in any suitable reactor e.g. fixed bed, fluid bed or spouted bed reactors. It is preferred to carry out the process in a fixed bed reactor. The process may also be carried out in two stages using a reactor comprising two zones wherein the first zone accommodates a reaction to provide a gaseous hydrocarbon and oxygen-containing gas. These products are then passed into the second zone with the paraffin-containing hydrocarbon feed stream to produce the olefin-containing product. This process is described in GB Patent Application 9819603.3 (BP Case 9051).

The products of the process of the present invention are suitably ethene, propene, butenes, pentenes and carbon monoxide. In addition to these products, aromatic hydrocarbon as well as small amounts of methane, acetylenes, water, hydrogen and carbon dioxide may be produced.

The product stream obtained from the autothermal cracking process is contacted with a solution of a metallic salt to isolate ethene and propene. Preferably prior to this step, the product stream is treated to remove components such as carbon dioxide, oxygen, hydrogen, oxygenates and the like which may be present.

The product stream is then contacted with a solution of a metallic salt capable of selectively chemically absorbing ethene and propene. Suitable metals salts are those capable of forming complexes with ethene and propene. The metallic salts may suitably comprise chromium, copper (I), manganese, nickel, iron, mercury, silver, gold, platinum, palladium, rhodium, ruthenium, osmium, molybdenum, tungsten and rhenium. The preferred metallic salt comprises copper (I) or silver.

Where the metallic salt is a copper (I) salt, the copper salt may be copper (I) acetate, copper (I) nitrate, copper (I) sulphate or copper carboxylates. Preferably the copper salt is copper (I) nitrate.

Where the metallic salt is a silver salt, the silver salts may be silver nitrate or silver fluoroborate.

The product stream from the autothermal cracking process is contacted with a solution of the metallic salt. Preferably the solution is an aqueous solution. The solution may also comprise an organic nitrogen-containing compound such as pyridine, piperidine, hydroxy-proprionitrile, diethylene triamine, acetonitrile, formamide and acetamide and derivatives thereof.

The concentration of metal salt to nitrogen-containing compound is the aqueous solution for copper (1) salt is suitably in the range 1:1 to 1:6, preferably 1:2.

The concentrations of metal salt in the aqueous solution is suitably at least 0.5 moles of salt per litre of solvent, preferably at least 2 moles of salt per litre of solvent.

The process for isolating the ethylene and propene comprises contacting the product stream obtained from the autothermal cracking process with a metal complex which is capable of complexing or absorbing ethylene and/or propylene. The separation process may be carried out by any suitable means known to the person skilled in the art. For example, the first stage of the separation process can be carried out in an absorber column designed to provide efficient contacting between the gas mixture and the complexing solution. Optionally, the absorption stage of the process can utilise a hydrophobic hollow fibre membrane. Suitably the complexing reaction is carried out at a temperature of from -10 to 300°C, preferably from 5 to 70°C. Suitably, the reaction is carried out under a pressure of from 1 to 70 barg preferably 3 to 20 barg.

A second step in the separation process is the removal of dissolved gases from the complex solution. This may be carried out by degassing at a temperature of from 0 to 80°C, preferably 15 to 35°C above the temperature of formation of the complex. Alternatively this step may be carried out under pressure of 2 to 98% of the absolute pressure used to form the complex, preferably 10 to 80% of the absolute pressure used to form the complex. Alternatively, a combination of reduced pressure and increased temperature may be used.

The third step is recovery of ethene and/or propene by dissociation of these gases from the metal complex. Typically this dissociation may be carried out at a temperature of from 0 to 80°C, preferably 15 to 35°C above the temperature used to remove dissolved gases. Alternatively, this step may be carried out under pressure at 2 to 98% of the absolute pressure used to remove the dissolved gases, preferably 10 to 80% of the absolute pressure used to remove the dissolved gases. Alternatively, a combination of reduced pressure and increased temperature may be used.

The regenerated olefin-free solution is then returned to the absorber column to complex more olefins.

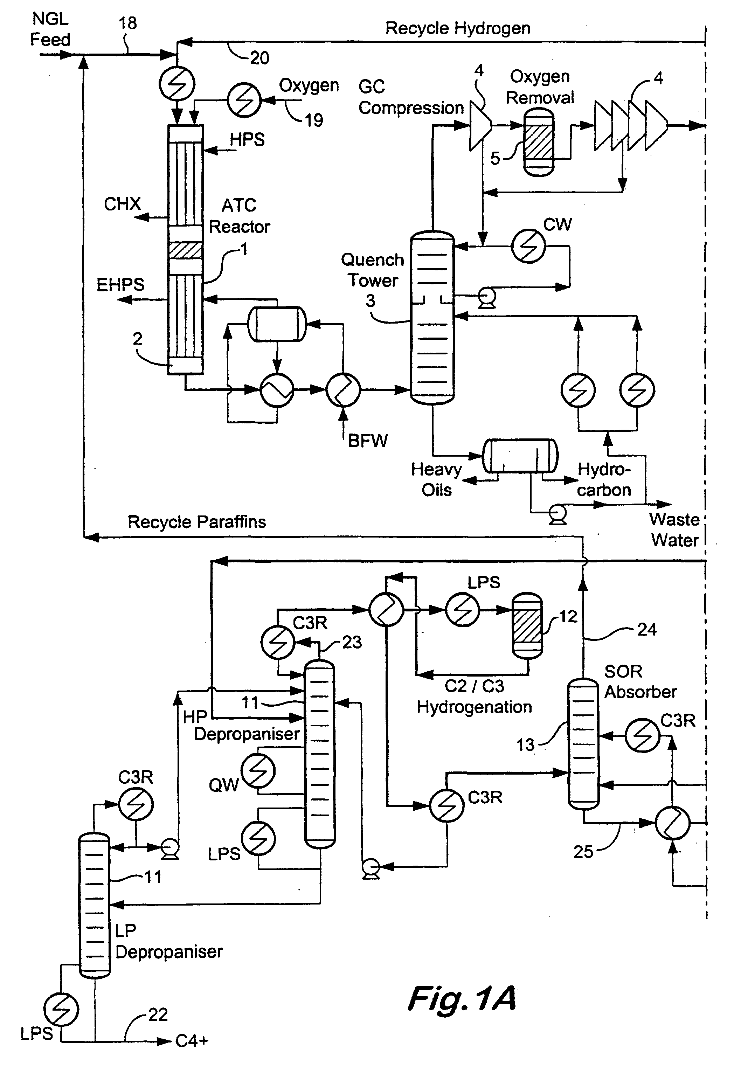

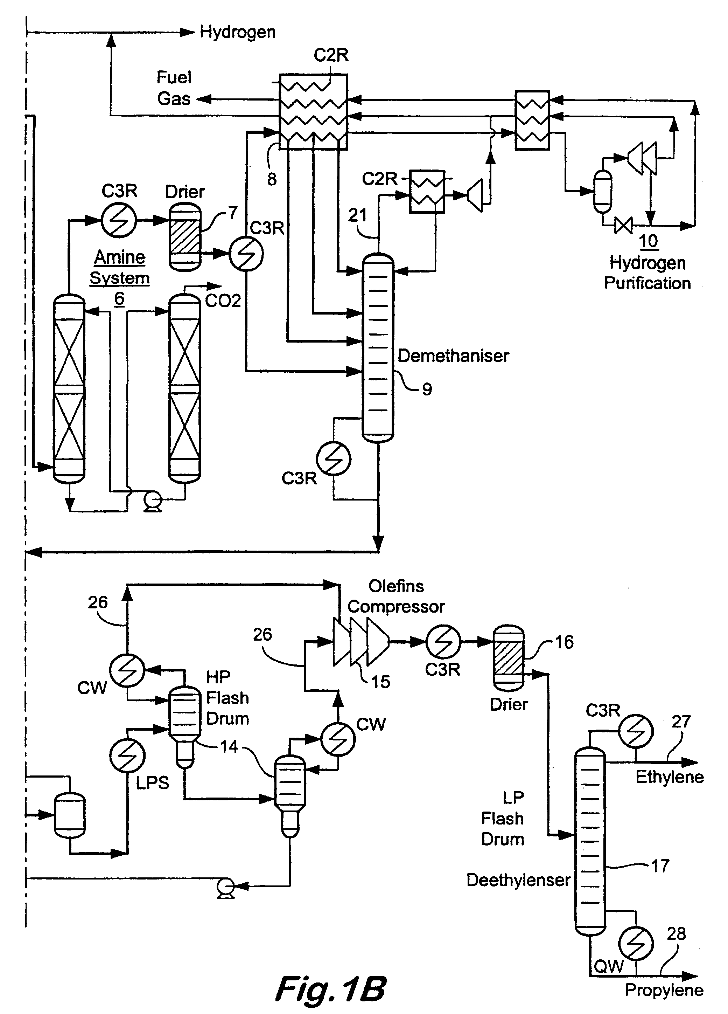

An embodiment of the invention will now be described by way of example with reference to the accompanying drawing, which is a schematic diagram of the apparatus employed to carry out an embodiment of the present invention.

The drawing depicts an apparatus comprising a reactor 1, which is coupled to an olefin absorber 13, via a series of purification units. The absorber 13 selectively absorbs ethene and propene produced in the reactor 1. The purification units include an oxygen removal unit 5, an amine system 6, a de-methaniser 9, and a depropaniser 11. Coupled to the absorber 13 is a de-ethyleniser 17, which isolates the ethene and propene produced in the reactor 1.

In operation, a paraffinic hydrocarbon feed 18, an oxygen-containing gas 19 and hydrogen 20 are fed to the reactor 1. The products from the reactor 1 comprise ethene, propene, water, carbon oxides, hydrogen, and other hydrocarbons.

The product stream is cooled using quench heat exchangers 2, and directed through a quench water tower 3, which removes most of the water from the stream. The stream is then compressed in compressor 4, and passed through the oxygen removal unit 5, where residual oxygen is removed. Once substantially free of oxygen, the stream is compressed again, using a second compressor 4. Carbon dioxide is then removed from the stream using the amine system 6.

Once substantially free of carbon dioxide, the stream is removed from the amine system 6, and dried using a molecular sieve dryer 7. The stream is then chilled in a pre-chilling system 8, and fed into the demethaniser 9. The pre-chilling system 8 and demethaniser 9 separate hydrogen, methane and carbon monoxide from the stream, as an overhead stream 21. The hydrogen from stream 21 is recovered using a hydrogen purification system 10 and recycled to the reactor 1 via line 20.

Substantially free of hydrogen, methane and carbon monoxide, the product stream is removed from the base of the demethaniser 9 and fed into the depropaniser 11. The depropaniser 1I removes heavier hydrocarbons 22 (such as C4 and heavier hydrocarbons) from the stream, as a base stream. The remainder of the stream is recovered from the top of the depropaniser 11. This overhead stream 23 comprises C2 and C3 hydrocarbons, as well as acetylene, propadiene and methyl acetylene impurities. These impurities are hydrogenated by passing the stream 23 through hydrogenator 12.

Once hydrogenated, the stream is introduced into the absorber 13. In the absorber, the stream is contacted with silver nitrate solution. The ethene and propene in the product stream react with the silver nitrate to form a silver nitrate/olefin complex. The paraffinic hydrocarbons in the product stream are not complexed, and these are recycled to the reactor 1 via line 24.

A solution 25 containing the silver nitrate/olefin complex is removed from the base of the absorber 13. The solution 25 is directed into a series of flash drums 14, where it is subjected to a reduced pressure. Under such conditions, the silver nitrate/olefin complex decomposes, releasing ethene and propene. These olefins are recovered as overhead streams 26.

The overhead streams 26 are directed into compressor 15, which compresses the olefins, before introducing them into a dryer 16. Once dried, the olefins are separated into ethene 27 and propene 28 using the de-ethyleniser 17.

## Claims

1. A process for the production and recovery of olefins from a hydrocarbon feed comprising one or more paraffins, said process comprising the steps of:

   (a) partially combusting a mixture of the hydrocarbon feed and a molecular oxygen-containing gas in a reaction chamber, said mixture having a ratio of hydrocarbon to oxygen of greater than the stoichiometric ratio required for complete combustion to carbon dioxide and water, to produce a product stream comprising ethene and propene,
   (b) contacting said product stream with a solution of a metallic salt capable of selectively chemically absorbing ethene and propene to produce a chemically absorbed olefin rich liquid stream.
   (c) recovering said ethene and/or propene from the metallic salt.

2. A process as claimed in claim 1, wherein the feed is a gaseous feed.

3. A process as claimed in claim 1, wherein the feed is a liquid feed.

4. A process as claimed in claim 3, wherein methane, ethane, propane, butane and mixtures thereof are cofed into the reactor.

5. A process as claimed in any preceding claim, wherein the amount of oxygen required for the process of the present invention is from 2 to 20% of the amount required for complete combustion to carbon dioxide and water.

6. A process as claimed in any preceding claim, wherein step a) is carried out in the presence of a catalyst capable of supporting combustion beyond the normal fuel rich limit of flammability.

7. A process as claimed in any preceding claim, wherein prior to step b), the product stream is treated to remove components such as carbon dioxide, oxygen, hydrogen, oxygenates and the like which may be present in low levels.

8. A process as claimed in any preceding claim, wherein the solution of a metallic salt employed in step b) comprises at least one of chromium, copper (I), manganese, nickel, iron, mercury, silver, gold, platinum, palladium, rhodium, ruthenium, osmium, molybdenum, tungsten and rhenium.

9. A process as claimed in claim 8, wherein the metallic salt is a copper (I) salt selected from the group consisting of: copper (I) acetate, copper (I) nitrate, copper (I) sulphate and copper carboxylate. .

10. A process as claimed in claim 8, wherein the metallic salt is a silver nitrate or silver fluoroborate.

11. A process as claimed in any preceding claim, wherein the solution of a metallic salt employed in step b) also comprises an organic nitrogen-containing compound selected from the group consisting of: pyridine, piperidine, hydroxy-proprionitrile, diethylene triamine, acetonitrile, formamide, acetamide and derivatives thereof.

12. A process as claimed in claim 11, wherein the ratio of the concentration of said metallic salt to said nitrogen-containing compound in the solution of the metallic salt of step b) is in the range 1:1 to 1:6.

13. A process as claimed in any preceding claim, wherein step c) is carried out by subjecting the chemically absorbed olefin rich liquid stream produced in step b) to heat, reduced pressure, or a combination thereof.

## Patentansprüche

1. Verfahren zur Herstellung und Gewinnung von Olefinen aus einer Kohlenwasserstoffbeschickung mit einem oder mehreren Paraffinen, wobei das Verfahren die Stufen umfasst, dass

(a) eine Mischung der Kohlenwasserstoffbeschickung und eines molekularen Sauerstoff enthaltenden Gases in einer Reaktionskammer teilweise verbrannt wird, wobei die Mischung ein Verhältnis von Kohlenwasserstoff:Sauerstoff hat, das größer ist als das stöchiometrische Verhältnis, das für die vollständige Verbrennung zu Kohlendioxid und Wasser erforderlich ist, um einen Produktstrom mit Ethen und Propen zu erzeugen,

(b) der Produktstrom mit einer Lösung eines Metallsalzes in Kontakt gebracht wird, das selektiv chemisch Ethen und Propen absorbieren kann, um einen Flüssigkeitsstrom, der reich an chemisch absorbierten Olefinen ist, zu erzeugen,

(c) Ethen und/oder Propen aus dem Metallsalz gewonnen wird.

2. Verfahren nach Anspruch 1, wobei die Beschickung eine gasförmige Beschickung ist.

3. Verfahren nach Anspruch 1, wobei die Beschickung eine flüssige Beschickung ist.

4. Verfahren nach Anspruch 3, wobei Methan, Ethan, Propan, Butan und Mischungen davon gleichzeitig in den Reaktor geführt werden.

5. Verfahren nach einem der vorhergehenden Ansprüche, wobei die Menge an Sauerstoff, die für das erfindungsgemäße Verfahren erforderlich ist, 2 bis 20% der Menge ist, die für die vollständige Verbrennung zu Kohlendioxid und Wasser erforderlich ist.

6. Verfahren nach einem der vorhergehenden Ansprüche, wobei Stufe a) in Gegenwart eines Katalysators durchgeführt wird, der die Verbrennung über die normale brennstoffreiche Grenze der Entflammbarkeit hinaus unterstützen kann.

7. Verfahren nach einem der vorhergehenden Ansprüche, wobei vor Stufe b) der Produktstrom behandelt wird, um Komponenten, wie Kohlendioxid, Sauerstoff, Wasserstoff, Sauerstoffverbindungen und dgl., die in niedrigen Anteilen enthalten sein können, zu entfernen.

8. Verfahren nach einem der vorhergehenden Ansprüche, wobei die Lösung eines Metallsalzes, die in Stufe b) angewendet wird, mindestens eines der Elemente Chrom, Kupfer(I), Mangan, Nickel, Eisen, Quecksilber, Silber, Gold, Platin, Palladium, Rhodium, Ruthenium, Osmium, Molybdän, Wolfram und Rhenium enthält.

9. Verfahren nach Anspruch 8, wobei das metallische Salz ein Kupfer(I)salz ist ausgewählt aus der Gruppe bestehend aus Kupfer(I)acetat, Kupfer(I)nitrat, Kupfer(I)sulfat und Kupfercarboxylat.

10. Verfahren nach Anspruch 8, wobei das Metallsalz ein Silbernitrat oder Silberfluorborat ist.

11. Verfahren nach einem der vorhergehenden Ansprüche, wobei die Lösung eines Metallsalzes, die in Stufe b) angewendet wird, auch eine organischen Stickstoff enthaltende Verbindung enthält ausgewählt aus der Gruppe bestehend aus Pyridin, Piperidin, Hydroxypropionitril, Diethylentriamin, Acetonitril, Formamid, Acetamid und Derivaten davon.

12. Verfahren nach Anspruch 11, wobei das Verhältnis der Konzentration des Metallsalzes zu der stickstoffhaltigen Verbindung in der Lösung des Metallsalzes von Stufe b) im Bereich von 1:1 bis 1:6 liegt.

13. Verfahren nach einem der vorhergehenden Ansprüche, wobei Stufe c) durchgeführt wird, indem der an chemisch absorbierten Olefinen reiche Flüssigkeitsstrom, der in Stufe b) erzeugt wurde, Hitze, vermindertem Druck oder einer Kombination davon ausgesetzt wird.

## Revendications

1. Procédé de production et de récupération d'oléfines à partir d'une charge d'hydrocarbure comprenant une ou plusieurs paraffines, ledit procédé comprenant les étapes consistant à :

(a) brûler partiellement un mélange de la charge d'hydrocarbure et d'un gaz contenant de l'oxygène moléculaire dans une chambre de réaction, ledit mélange ayant un rapport de l'hydrocarbure à l'oxygène supérieur au rapport stoechiométrique requis pour la combustion complète en dioxyde de carbone et en eau, pour produire un flux de produit comprenant de l'éthène et du propène,

(b) amener ledit flux de produit au contact d'une solution d'un sel métallique capable d'absorber chimiquement et sélectivement l'éthène et le propène pour produire un flux de liquide riche en oléfines chimiquement absorbées,

(c) récupérer ledit éthène et/ou propène à partir du sel métallique.

2. Procédé selon la revendication 1, dans lequel la charge est une charge gazeuse.

3. Procédé selon la revendication 1, dans lequel la charge est une charge liquide.

4. Procédé selon la revendication 3, dans lequel du méthane, de l'éthane, du propane, du butane et des mélanges de ceux-ci sont amenés conjointement dans le réacteur.

5. Procédé selon l'une quelconque des revendications précédentes, dans lequel la quantité d'oxygène requise pour le procédé de la présente invention est de 2 à 20 % de la quantité requise pour la combustion complète en dioxyde de carbone et en eau.

6. Procédé selon l'une quelconque des revendications précédentes, dans lequel on effectue l'étape a) en présence d'un catalyseur capable d'entretenir la combustion au-delà de la limite normale d'inflammabilité d'un mélange riche en combustible.

7. Procédé selon l'une quelconque des revendications précédentes, dans lequel, avant l'étape b), on traite le flux de produit pour séparer des composants tels que du dioxyde de carbone, de l'oxygène, de l'hydrogène, des composés oxygénés et analogues qui peuvent être présents en petites quantités.

8. Procédé selon l'une quelconque des revendications précédentes, dans lequel la solution d'un sel métallique employée dans l'étape b) comprend au moins un des éléments chrome, cuivre (I), manganèse, nickel, fer, mercure, argent, or, platine, palladium, rhodium, ruthénium, osmium, molybdène, tungstène et rhénium.

9. Procédé selon la revendication 8, dans lequel le sel métallique est un sel de cuivre (I) choisi dans l'ensemble constitué de : acétate de cuivre (I), nitrate de cuivre (I), sulfate de cuivre (I) et carboxylate de cuivre.

10. Procédé selon la revendication 8, dans lequel le sel métallique est un nitrate d'argent ou un fluoroborate d'argent.

11. Procédé selon l'une quelconque des revendications précédentes, dans lequel la solution d'un sel métallique employée dans l'étape b) comprend aussi un composé organique contenant de l'azote, choisi dans l'ensemble constitué de : pyridine, pipéridine, hydroxyproprionitrile, diéthylènetriamine, acétonitrile, formamide, acétamide et des dérivés de ceux-ci.

12. Procédé selon la revendication 11, dans lequel le rapport de la concentration dudit sel métallique audit composé contenant de l'azote dans la solution de sel métallique de l'étape b) est compris dans la plage de 1:1 à 1:6.

13. Procédé selon l'une quelconque des revendications précédentes, dans lequel on effectue l'étape c) en soumettant le flux de liquide riche en oléfines chimiquement absorbées, produit dans l'étape b), à la chaleur, à une pression réduite ou à une combinaison de celles-ci.

Fig.1A

*Fig.1B*